# EUROPÄISCHE PATENTSCHRIFT

(11) **EP 0 842 926 B1**
(45) Veröffentlichungstag und Bekanntmachung des Hinweises auf die Patenterteilung: **13.02.2002**
(21) Anmeldenummer: 97118891.7
(22) Anmeldetag: 30.10.1997
(51) Int. Cl.: C07D 309/04

(54) **Verfahren zur Herstellung von Rosenoxid**
Process for the preparation of rose oxide
Procédé pour la préparation de l'oxyde de rose

(30) Priorität: 08.11.1996 DE 19645922
(43) Veröffentlichungstag der Anmeldung: 20.05.1998
(73) Patentinhaber: Dragoco Gerberding & Co Aktiengesellschaft, 37603 Holzminden (DE)
(72) Erfinder: Pickenhagen, Wilhelm, Dr., 37671 Höxter (DE); Schatkowski, Dietmar, 37627 Stadtoldendorf (DE)
(74) Vertreter: Eisenführ, Speiser & Partner

(56) Entgegenhaltungen:
- EP-A- 0 761 628
- CH-A- 503 723
- DE-A- 1 443 338
- DE-B- 1 137 730
- J. S. PATEL ET AL.: INDIAN JOURNAL OF CHEMISTRY, Bd. 16b, Nr. 3, 1978, Seiten 188-90, XP002053576
- G. OHLOFF ET AL.: ANGEWANDTE CHEMIE, Bd. 73, Nr. 16, 1961, Seite 578 XP002053577

## Beschreibung

### Verfahren zur Herstellung eines Isomerengemisches von Z- und E-2-[2-Methyl-prop-1-en-1-yl-]-4-methyl-tetrahydropyran der allgemeinen Formel A

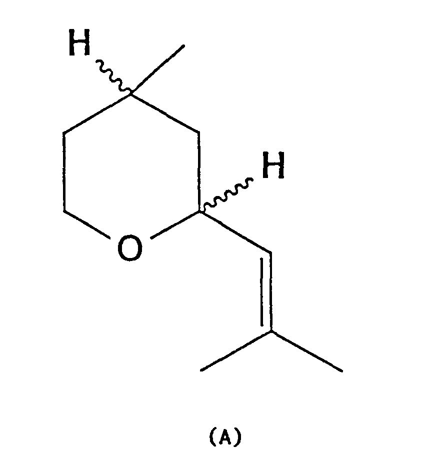

Die Erfindung betrifft ein verbessertes Verfahren zur Herstellung eines Isomerengemisches von Z- und E-2-[2-Methyl-prop-1-en-1-yl-]-4-methyl-tetrahydropyran der allgemeinen Formel **A**, besser unter dem Namen cis- und trans-Rosenoxid bekannt, das in der Regel mindestens 80 % des für die Parfümerie wertvolleren natürlichen Z-Isomeren (cis-Rosenoxid) enthält. Dieses Isomerengemisch wird nachfolgend auch "Rosenoxid der allgemeinen Formel **A**" genannt; dieser Begriff umfaßt racemische ebenso wie optisch aktive Isomerengemische.

Der allgemeinere Begriff "Rosenoxid" umfaßt im Rahmen dieser Anmeldung neben dem erfindungsgemäßen Isomerengemisch ("Rosenoxid der allgemeinen Formel **A**") mit in der Regel mindestens 80% des Z-Isomeren auch andere Gemische des Z- und des E-Isomeren sowie die reinen Isomere und Enantiomere.

Seit der Isolierung [C. Seidel, M. Stoll, Helv. Chim. Acta, 42, 1830 (1959); Y. Naves, D. Lamparsky, P. Ochsner, Bull. Soc. Chim. Fr., 645 (1961)] und Strukturaufklärung [Y. Naves, D. Lamparsky, P. Ochsner, Bull. Soc. Chim. Fr., 645 (1961); C. Seidel, D. Felix, A. Eschenmoser, K. Bieman, E. Palluy und M. Stoll, Helv. Chim. Acta, 44, 598 (1961)] des Rosenoxids erfolgten zahlreiche Veröffentlichungen, die sich auf neue Syntheseverfahren zu seiner Herstellung bezogen.

So wird in der EP 0021769 und in Tetrahedron Letters 39, 3599-3602 (1971) von T. Schono, A. Ikeda, Y. Kimura die elektrochemische Darstellung von optisch aktivem bzw. racemischem Rosenoxid, ausgehend von optisch aktivem bzw. racemischem Citronellol beschrieben.

Weitere Synthesen gehen von 3-Methyl-but-2-en-1-al und 2-Methyl-but-1-en-4-ol [J.H.P. Tyman, B.J. Willis, Tetrahedron Letters 51, 4507 (1970)] und Epoxy-β-Citronellol [G. Ohloff, B. Lienhardt, Helv. Chim. Acta 182-189 (1964)] aus.

In der DE 3150234 wird über ein Verfahren zur Herstellung eines Gemisches aus mindestens 80 % cis- und höchstens 20 % trans-Rosenoxid berichtet, wobei das Verfahren darin besteht, daß man 2-[2-Methyl-prop-1-en-1-yl]-4-methylen-tetrahydropyran an einem Platindioxid- oder einem Platin/Kohlekatalysator in Gegenwart eines stark sauren Kationenaustauschers hydriert.

G. Ohloff, E. Klein, G. Schade werden in der DE 1137730 und DE 1443338 der Studiengesellschaft Kohle mbH als Erfinder eines Verfahrens genannt, welches (+)- und (-)- sowie racemisches Citronellol (**1**) durch photosensibilisierte Singulett-Sauerstoff-Oxidation in ein Gemisch zweier Peroxide (**2a/b**) überführt und diese in an sich bekannter Weise [L.-F. Tietze, Th. Eicher, 428-430, (1981), Jord. Stein Verlag, Stuttgart, New York] mit Na₂SO₃ zu einem Gemisch der beiden Diole **3a/b** reduziert. Durch Behandlung dieses Gemisches mit verdünnten Säuren wird nur **3a** (Schema 1) in ein Gemisch der cis- und trans-Rosenoxide **4** überführt. Dieses Verfahren wird von G. Ohloff, E. Klein und G.O. Schenck in der Angew. Chem. 73, 578 (1961) nochmals ausführlich beschrieben. Dabei erhält man, je nach Wahl der entsprechenden Citronellole, ausgehend von (-)-Citronellol die (-)-Rosenoxide, von (+)-Citronellol die (+)-Rosenoxide und ausgehend von racemischem (+/-)-Citronellol das Gemisch der cis-/trans-isomeren optisch inaktiven Rosenoxide. Bei der photosensibilisierten Umsetzung von Singulett-Sauerstoff mit Citronellol reagieren die Wasserstoffatome beider Kohlenstoffe der Isopropyliden-Doppelbindung praktisch gleich schnell, so daß (nach anschließender Na₂SO₃- Reduktion) ein nahezu 1:1-Gemisch der beiden Diole (**3a**) und (**3b**) entsteht.

Seit den ersten Untersuchungen über die Umwandlung des Diol-Gemisches **3a/b** in Rosenoxid **4**, wurde immer wieder darauf hingewiesen [G. Ohloff, B. Lienhardt, Helv. Chim. Acta 182-189 (1964); L.-F. Tietze, Th. Eicher, 428-430, (1981), Jord. Stein Verlag, Stuttgart, New York; G. Ohloff, Pure Applied Chem., 481-502, (1975)], daß sich nur das Diol **3a** in Rosenoxid überführen läßt.

Nach G. Ohloff, E. Klein und G.O. Schenck, Angew. Chem. 73, 578 (1961) läßt sich das Diol (**3b**) bei Zimmertemperatur nicht verändern, wohl aber bei höheren Temperaturen mittels starker Säuren. Es wird dann ein Oxidgemisch erhalten, das neben den Rosenoxiden (cis- und trans-Rosenoxid) hauptsächlich Isomere mit der Isopropenylgruppe, sogenannte Iso-Rosenoxide (**B**) enthält.

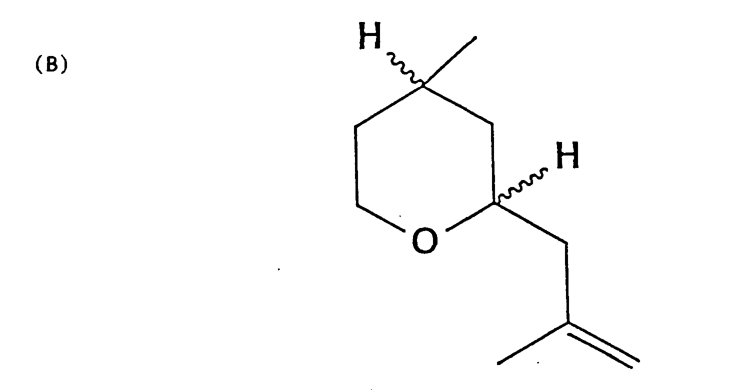

Das bekannte Verfahren, die Herstellung von optisch aktivem oder racemischem Rosenoxid durch
- photosensibilisierte Singulett-Sauerstoff-Oxidation von (+)- oder (-)- oder (+/-)-Citronellol,
- Reduktion des erhaltenen Hydroperoxidgemisches (**2a+2b**) zu dem Diolgemisch (**3a+3b**) und
- sich daran anschließende, gemäß bekannter Verfahrensweise [L.-F. Tietze, Th. Eicher, 428-430, (1981), Jord. Stein Verlag, Stuttgart, New York] säurekatalysierte Cyclisierung zum cis-/trans-Rosenoxid (Schema 1 - Verbindung **4**),
ist für eine technische Anwendung also nicht zufriedenstellend, da sich das nach diesem Verfahren im zweiten Verfahrensschritt zu 40 - 45 % anfallende 3,7-Dimethyl-oct-7-en-1,6-diol (**3b**) gar nicht oder nur unter drastischen Bedingungen, wobei dann erhebliche Anteile vom sogenannten Iso-Rosenoxid (**B**) entstehen, in Rosenoxid überführen läßt, vergleiche oben [G. Ohloff, E. Klein, G.O. Schenck, Angew. Chem. 73, 578 (1961)].

Die bekannte Vorgehensweise [L.-F. Tietze, Th. Eicher, 428-430, (1981), Jord. Stein Verlag, Stuttgart, New York] liefert in ca. 39 %iger Ausbeute die entsprechenden Rosenoxide; eine Umsetzung des Diols (**3b**) findet jedoch nicht (oder nur in vernachlässigbarem Umfang) statt.

Entsprechend dem beschriebenen Stand der Technik ist es daher besonders überraschend, daß das erfindungsgemäße Verfahren (mit Varianten A, B, C, D) welches durch das **Schema 2** veranschaulicht und weiter unten näher erläutert wird, die Umwandlung von 3,7-Dimethyl-oct-7-en-1,6-Diol (**3b**) einzeln oder im Gemisch mit 3,7-Dimethyl-oct-5-en-1,7-diol (**3a**) ins Rosenoxid (**5a/b**) [Z-/E-2-(2-Methyl-prop-1-en-1-yl)-4-methyl-tetrahydropyran] ermöglicht. Das Rosenoxid (**5a/b**) entspricht dabei dem Rosenoxid der allgemeinen Formel **A**.

Bei dem erfindungsgemäßen Verfahren zur Herstellung von Rosenoxid der allgemeinen Formel **A** wird 3,7-Dimethyl-oct-7-en-1,6-diol (**3b**) in Anwesenheit eines gegebenenfalls in situ gebildeten Allylethers mit Säure behandelt, wobei die Säurebehandlung in einem Zweiphasensystem flüssig/ flüssig unter den Bedingungen einer Phasentransferkatalyse oder in einem Zweiphasensystem flüssig/fest mittels einer an einer Festsubstanz gebundenen Säure erfolgt. Die reaktionsmechanistische Funktion des Allylethers ist dabei momentan noch nicht geklärt, seine Anwesenheit aber essentiell zum Erreichen einer hinsichtlich der Ausbeute zufriedenstellenden Umsetzung des Diols (**3b**) zu Rosenoxid.

Das erfindungsgemäße Verfahren führt zu Isomerengemischen, die - wie oben erwähnt - in der Regel mindestens 80 % des für die Parfümerie wertvolleren natürlichen Z-Isomeren (cis-Rosenoxid) enthalten; häufig werden jedoch sogar Isomerengemische mit 90% oder mehr cis-Rosenoxid erreicht, dies ist natürlich besonders vorteilhaft.

Das erfindungsgemäße Verfahren führt zu guten Resultaten, unabhängig davon, ob 3,7-Dimethyl-oct-7-en-1,6-diol (**3b**) einzeln oder im Gemisch mit 3,7-Dimethyl-oct-7-en-1,7-diol (**3a**) in Rosenoxid überführt wird.

Vorzugsweise handelt es sich bei dem erfindungsgemäß eingesetzten Allylether selbst um ein Rosenoxid; dieses wird mit dem 3,7-Dimethyl-oct-7-en-1,6-diol (**3b**) oder dem Diol-Gemisch (**3a + 3b**) vor oder während der erfindungsgemäßen Säurebehandlung vermischt und/oder bildet sich während der Säurebehandlung in situ aus dem gegebenenfalls anwesenden 3,7-Dimethyl-oct-7-en-1,7-diol (**3a**). In Gegenwart eines Rosenoxids werden besonders gute Ausbeuten bei der Umsetzung des Diols (**3b**) zu Rosenoxid der allgemeinen Formel A erzielt. Vorteilhafterweise wird eine Stoffmenge des 3,7-Dimethyl-oct-7-en-1,6-diols (**3b**), gegebenfalls im Gemisch mit dem Diol (**3a**), mit einer gleichen, größeren oder nur geringfügig geringeren Stoffmenge Rosenoxid vermischt. Ein solcher externer Zusatz von Rosenoxid in überkatalytischer Menge führt häufig zu besonders guten Ausbeuten.

In der Regel wird das erfindungsgemäße Verfahren bei erhöhter Temperatur durchgeführt. In einer Vielzahl von Fällen ist es günstig, die Reaktiontemperatur bei der Säurebehandlung zumindest zeitweise auf die Siedetemperatur der flüssigen Phase einzustellen, im Zweiphasensystem flüssig/flüssig auf die Siedetemperatur der niedriger siedenden flüssigen Phase.

Zur Säurebehandlung des Diol-Gemisches (**3a + 3b**) oder des Diols (**3b**) kann beispielsweise Schwefelsäure, Phosphorsäure, eine säureaktivierte Bleicherde oder eine Heteropolywolframsäure wie Wolframatokieselsäure oder Wolframatophosphorsäure eingesetzt werden.

Wird die erfindungsgemäße Säurebehandlung in einem Zweiphasensystem flüssig/ fest mittels einer an einer Festsubstanz gebundenen Säure durchgeführt, so ist es in der Regel günstig, eine Behandlung mit handelsüblichen säureaktivierten Bleicherden wie Montmorillonit K10, Montmorillonit KSF, Filtrol, Katalysator KS, Tonsil Optimum und ähnlichen am Markt verfügbaren Produkten in Konzentrationen von 0,5 bis 40 Gew.%, vorzugsweise von 1 - 20 Gew.%, bezogen auf eingesetztes Diolgemisch (**3a + 3b**) bzw. Diol (**3b**) vorzusehen.

Wird die erfindungsgemäße Säurebehandlung in einem Zweiphasensystem flüssig/ flüssig unter den Bedingungen einer Phasentransferkatalyse durchgeführt, so können insbesondere Phasentransferkatalysatoren wie z.B. Aliquat, Tetrabutylammonium-chlorid, -bromid oder hydrogensulfat eingesetzt werden, beispielsweise in Konzentrationen von 0,1 Gew.-% bis 10 Gew.-%, vorzugsweise von 4-6 Gew.-%, bezogen auf die Menge des eingesetzten Diolgemischs (**3a + 3b**) oder Diols (**3b**).

Wird in dem erfindungsgemäßen Verfahren ein Gemisch der beiden Diole (**3a**) und (**3b**) eingesetzt, so ist dieses vorzugsweise aus Citronellol der allgemeinen Formel (**1**) z.B. durch photosensibilisierte Singulett-Sauerstoff-Oxidation und anschließende Reduktion der erhaltenen Hydroperoxide (**2a + 2b**) gewonnen, vgl. **Schema 2**. Insoweit können die bekannten Verfahren eingesetzt werden (siehe oben und Schema 1).

Es ist zur Durchführung des erfindungsgemäßen Verfahrens vorteilhaft, das Diolgemisch (**3a + 3b**) oder das Diol (**3b**) in einem aprotischen Lösungsmittel zu lösen. Als aprotische Lösungsmittel kommen insbesondere Pentan, Hexan, Heptan, Cyclohexan, Benzol, Toluol oder Xylol sowie ähnliche handelsübliche Kohlenwasserstoffe und Gemische dieser Lösungsmittel in Frage.

Die Testung der sensorischen Eigenschaften von Iso-Rosenoxid (**B**) und Gemischen von **B** mit Rosenoxid (**5a/b**), wobei **5a/b** in größeren Anteilen **B** enthält, ergab, daß das Iso-Rosenoxid (**B**) weniger wünschenswerte sensorische Eigenschaften besitzt. Iso-Rosenoxid (**B**) zeichnet sich durch fettig-terpenige, wenig krautige Aspekte aus. Ein Vorhandensein in Anteilen > 2 % im Cyclisierungsprodukt führt daher zur Beeinträchtigung bei Verwendung solcher Gemische als Riechstoff. G. Ohloff (G.Ohloff, Olfaction and Taste, 4, 156 (1972) und H. Medsuda et al. (H. Medsuda et al., Flavors, Fragrances and Essential Oils, 3, 85-91, (1995)] beschreiben im einzelnen die sensorischen Eigenschaften der 4 optischen Antipoden des Rosenoxids. Beide kommen zu dem Schluß, daß es sich bei den entsprechenden cis-Verbindungen (**5a**) um die sensorisch stärkeren und interessanteren Verbindungen handelt.

Dem erfindungsgemäßen Verfahren kommt nun zugute, daß man das Diol **3b** sowohl in Mischung mit als auch getrennt vom Diol **3a** je nach Wahl der Reaktionsbedingungen unter deutlicher Ausbeutesteigerung in nur einer Stufe in ein mindestens 80 %iges cis-Rosenoxid (**5a**) überführen kann. Die Bildung des sensorisch unerwünschten Iso-Rosenoxids (**B**) erfolgt sich bei den nachfolgend aufgeführten Verfahrens-Varianten A, B, C, D (Beispiele 3-6) im Bereich < 2 %.

Die nachfolgenden Beispiele erläutern die Erfindung, ohne sie zu beschränken. Die ermittelten Ausbeuten der Rosenoxide wurden dabei auf eingesetztes (-)-Citronellol, (+)-Citronellol und racemisches (±)-Citronellol bezogen.

Die Beispiele 1 und 2 betreffen bevorzugte Verfahrensschritte zur Herstellung von racemischem Citronellyl-Hydroperoxid (**2a/b**) und racemischem Diol-Gemisch (**3a/b**).

Die Beispiele 3 - 6 (Verfahrens-Varianten A-D) betreffen jeweils eine erfindungsgemäße Herstellung von racemischem cis/trans-Rosenoxid (**5a + 5b**) aus dem gemäß Beispiel 2 hergestellten Diol-Gemisch **3a/b**.

Beispiel 7 betrifft die Herstellung eines Gemischs aus optisch aktivem (-)-3,7-Dimethyl-oct-5-en-1,7-diol und (-)-3,7-Dimethyl-oct-7-en-1,6-diol; Beispiel 8 betrifft die von diesem Gemisch ausgehende erfindungsgemäße Herstellung von (-)-cis/trans-Rosenoxid.

Die Beispiele 9 und 10 betreffen die entsprechende erfindungsgemäße Herstellung eines Gemischs aus optisch aktivem (+)-3,7-Dimethyl-oct-5-en-1,7-diol und (+)-3,7-Dimethyl-oct-7-en-1,6-diol sowie die von diesem Gemisch ausgehende erfindungsgemäße Herstellung von (+)-cis/trans-Rosenoxid.

Beispiel 11 betrifft die Herstellung des (unvermischten) Diols 3,7-Dimethyl-oct-7-en-1,6-diol (**3b**).

Beispiel 12 ist ein nicht-erfindungsgemäßes (Vergleichs-)Beispiel zur Herstellung von racemischem cis/trans-Rosenoxid aus dem unvermischten Diol (**3b**) gemäß Beispiel 11; die Beispiele 13 und 14 sind hingegen bevorzugte erfindungsgemäße Beispiele zur Herstellung des racemischen cis/trans-Rosenoxid aus dem unvermischten Diol (**3b**), wobei die Verfahrens-Ausbeute gemäß Beispiel 14 noch deutlich höher ist als die gemäß Beispiel 13.

### Beispiel 1

### Herstellung von racemischem Citronellyl-Hydroperoxid (2a/b)

In einer Belichtungsapparatur aus Quarz-Glas mit einem Gaseinleitungsrohr (Fritte) und Rückflußkühler werden 112 g (0,72 mol) racemisches Citronellol synthetisch, 300 ml Methanol und 500 mg Rose Bengale unter Einleiten eines Sauerstoffstroms (ca. 700 ml/h) über einen Zeitraum von 15 - 16 h bei 20 - 25 °C mit einem 150 Watt Quecksilber-Hochdruckbrenner bestrahlt.

Man erhält eine Photooxidationslösungmit racemischem Citronellyl-Hydroperoxid (**2a/b**).

### Beispiel 2

### Herstellung von racemischem Diol-Gemisch (3a/b)

In einem 1 l-Dreihalskolben mit Rückflußkühler und Tropftrichter werden 100,8 g (0,8 mol) Na₂SO₃ und 300 ml Wasser vorgelegt. Dann tropft man innerhalb von 1 h bei 50 °C die unter Beispiel 1 (**2a/b**) hergestellte Photooxidationslösung zu. Anschließend wird über einen Zeitraum von 3 - 4 h bei 40 - 50 °C nachgerührt, und über einen Zeitraum von 1 h ca. 550 ml Methanol/Wasser abdestilliert. Nach dem Abkühlen auf Raumtemperatur wird mit H₂O versetzt, kräftig verrührt und die wäßrige Phase nach dem Absetzen abgetrennt.

Es verbleiben 150 g Rohprodukt (racemisches Diol-Gemisch (**3a/b**)).

Gaschromatogramm (Shimadzu GC 14A, DB-1, 30 m, 100 - 240 °C, 10 °C/min);
**3a** Rₜ = 7,6'; (46 %)
**3b** Rₜ = 8,5'; (45 %)

### Beispiel 3

### Herstellung von racemischem cis/trans-Rosenoxid (5a + 5b) [9:1]

### Variante A

In einem 2 l-Dreihalskolben mit Tropftrichter, Thermometer, Rückflußkühler und Wasserabscheider werden 50 g (0,27 mol) Diol-Gemisch **3a/b** (aus Beispiel 2), 1,5 Hexan 63/80 und 2,5 g Filtrol® über einen Zeitraum von 10 h unter Rückfluß gerührt. Während dieser Zeit werden insgesamt 4,8 ml Wasser abdestilliert.

Nach dem Abkühlen auf Raumtemperatur wird das Filtrol abfiltriert und die org. Phase mit Sodalösung und Wasser neutralgewaschen. Nach Abdestillation des Lösungsmittels verbleiben 39 g Rohprodukt (racemisches cis/trans-Rosenoxid (**5a + 5b**).

GC (Bedingungen siehe Beispiel 2): **5a** (66,9 %); **5b** (7,4 %); **B** = 1,4 %
Σ **5a + 5b** = 74,3 %
**5a : 5b** = 90:10

Eine Destillation über eine 30 cm Metallfüllörperkolonne ergab 22,1 g KP_{22mbar} = 80° - 81 °C. Das entspricht einer Ausbeute von 59,2 %.
GC (Bedingungen siehe Beispiel 2): **5a** (90,1 %); **5b** (7,9 %); **B** = 0,3 %
Σ **5a + 5b** = 98,0 %
**5a : 5b** = 92:8
- D 20/4 =: 0,8703
- n 20/D =: 1,4539
- [α] 20/D =: 0,1°

GC/MS: HP 5970 B, DBWax-60 N, 60 m, 60 - 240 °C, 4 °C/min
**5a** Rₜ = 15,5
MS (70 eV): m/e (%) = 154 (16, M⁺), 139 (100), 85 (13), 83 (28), 69 (63), 67 (11), 55 (28),
39 (15).

**5a** ¹³C-NMR (CDCl₃), Varian VXR-300: δ [ppm]: 18.34, 22.32, 25.70 (CH₃), 34.43, 40.82,
67.88 (CH₂), 30.25, 74.62, 126.37 (CH), 135.05 (C).

5 b Rₜ = 16.09
MS (70 ev): m/e (%) = 154 (11, M⁺), 139 (100), 85 (11), 83 (26), 69 (63), 67 (11), 55 (29),
39 (15).

**5b** ¹³C-NMR (CDCl₃), Varian VXR-300: δ [ppm] = 18.27, 19.23, 25.79 (CH₃), 32.54, 38.21, 62.13 (CH₂ ), 24.97, 69.10, 125.37 (CH), 135.48 (C).

**B** Rₜ = 15.12
MS (70 eV): m/e (%) = 154 (35, M⁺), 139 (100), 83 (22), 71 (50), 69 (61), 67 (23), 55 (64),
41 (79).

Rₜ = 15,20
MS (70 eV): m/e (%) = 154 (37, M⁺), 139 (100), 83 (23), 71 (49), 69 (68), 67 (25), 55 (62),
41 (80).

### Beispiel 4

### Herstellung von racemischem cis/trans-Rosenoxid (5a + 5b) [9:1]

### Variante B

In einem 1 l-Rührwerk mit Tropftrichter, Thermometer und Rückflußkühler werden 300 g Toluol, 50 g 50 %ige Schwefelsäure, 1 g Aliquat® R 336 und 50 g (0,27 mol) Diol-Gemisch **3a/b** (aus Beispiel 2) 15 Minuten unter Rückfluß gerührt, auf Raumtemperatur abgekühlt, die organische Phase abgetrennt, mit Sodalösung und Wasser neutralgewaschen, über Natriumsulfat getrocknet und das Lösungsmittel unter vermindertem Druck abdestilliert.

Es verbleiben 42 g Rohprodukt (racemisches cis/trans-Rosenoxid (**5a + 5b**)).

GC (Bedingungen siehe Beispiele 2): **5a** (66,2 %); **5b** (5,9 %); B = 0,4 %
Σ **5a + b** = 72,4 %
**5a : 5b** = 91:9

Eine Destillation über eine 30 cm Metallfüllörperkolonne ergab 22,3 g KP_{22mbar} = 80° - 81,5 °C. Das entspricht einer Ausbeute von 59,8 %.
GC (Bedingungen siehe Beispiel 2): **5a** (90,0 %); **5b** (7,8 %); **B** = 0,35 %
Σ **5a + 5b** = 97,8 %
**5a : 5b** = 92:8
- D 20/4 =: 0,8699
- n 20/D =: 1,4540
- [a] 20/D =: 0,1°

GC/MS: Bedingungen siehe Beispiel 3

**5a** Rₜ = 15,56
MS (70 eV): m/e (%) = 154 (12, M⁺), 139 (100), 85 (11), 83 (24), 69 (58), 67 (11), 55 (22),
41 (23), 39 (14).

**5b** Rₜ = 16,09
MS (70 eV): m/e (%) = 154 (10, M⁺), 139 (100), 85 (9), 83 (22), 69 (53), 57 (10), 55 (18),
41 (18), 39 (11).

**5a** ¹³C-NMR (CDCl₃), Varian VXR-300: δ [ppm]: 18.35, 22.33, 25.70 (CH₃), 34.40, 40.83, 67.88 (CH₂), 30.27, 74.62, 126.38, (CH), 135.05 (C).

**5b** ¹³C-NMR (CDCl₃), Varian VXR-300: δ [ppm]: 18.28, 19.24, 25.78 (CH₃), 32.54, 38.20, 62.11 (CH₂), 24.97, 69.10, 125.36 (CH), 135.45 (C).

**B** Rₜ = 15,12
MS (70 eV): m/e (%) = 154 (35, M⁺), 139 (100), 83 (21), 71 (49), 69 (60), 67 (23), 55 (64),
41 (78).
Rₜ = 15,21
MS (70 eV): m/e (%) = 154 (37, M⁺), 139 (100), 83 (22), 71 (50), 69 (68), 67 (24), 55 (63),
41 (80).

### Beispiel 5

### Herstellung von racemischem cis/trans-Rosenoxid (5a + 5b) [9:1]

### Variante C

In einem 1 l-Rührwerk mit Tropftrichter, Thermometer, Wasserabscheider und Rückflußkühler werden 600 ml Cyclohexan und 50 g (0,27 mol) Diolgemisch **3a/b** (aus Beispiel 2) unter Rückfluß gerührt. Anschließend wird über einen Zeitraum von 2 h portionsweise insgesamt 5 g (1,74 mmol) Wolframatokieselsäurehydrat zugegeben und weitere 2 h unter Rückfluß nachgerührt. Während dieser Zeit werden insgesamt 4,3 ml Wasser abdestilliert. Nach dem Abkühlen auf Raumtemperatur wird mit Sodalösung neutralisiert und mit Wasser nachgewaschen. Nach dem Trocknen über Na₂SO₄ wird das Lösungsmittel unter vermindertem Druck abdestilliert.

Es verbleiben 40 g Rohprodukt (racemisches cis/trans-Rosenoxid (**5a + 5b**)).

GC (Bedingungen siehe Beispiel 2): **5a** (62,9 %); **5b** (6,0 X); **B** = 0,9 %
Σ **5a + 5b** = 68,9 %
**5a : 5b** = 91:9

Destillation über eine 30 cm Metallfüllkörperkolonne ergab 21,9 g Kp_{20mbar} 79 - 81 °C.
Das entspricht einer Ausbeute von 58,7 %.

GC (Bedingungen siehe Beispiel 2): **5a** (90.5 %); **5b** (8.1 %); **B** = 0,25 %
Σ **5a + 5b** = 98,6 %
**5a : 5b** = 92:8
- D 20/4 =: 0,8707
- n 20/D = 1,4550
- [α] 20/D =: 0,0°

GC/MS: Bedingungen siehe Beispiel 3

**5a** Rₜ = 15,59
MS (70 eV): m/e (%) = 154 (12, M⁺), 139 (100), 85 (10), 83 (23), 69 (55), 67 (10), 55 (21),
41 (21), 39 (13).

**5b** Rₜ = 16,09
MS (70 eV): m/e (%) = 154 (10, M⁺), 139 (100), 85 (10), 83 (23), 69 (59), 67 (12), 55 (22),
41 (22), 39 (12)

**5a** ¹³C-NMR (CDCl₃), Varian VXR-300: δ [ppm]: 18.35, 22.34, 25.70 (CH₃), 34.46, 40.85, 67.87 (CH₂), 30.29, 74.62, 126.44 (CH), 134.95 (C).

**5b** ¹³C-NMR (CDCl₃), Varian VXR-300: δ [ppm]: 18.27, 19.23, 25.78 (CH₃), 32.54, 38.20, 62.11 (CH₂), 24.97, 69.10, 125.36 (CH), 135.44 (C).

**B** Rₜ = 15,12
MS (70 eV): m/e (%) = 154 (35, M⁺), 139 (100), 83 (22), 71 (50), 69 (61), 67 (23), 55 (64), 41 (79).

Rₜ = 15,21
MS (70 eV): m/e (%) = 154 (37, M⁺), 139 (100), 83 (23), 71 (49), 69 (68), 67 (25), 55 (62), 41 (80).

### Beispiel 6

### Herstellung von racemischen cis/trans-Rosenoxid (5a/b) [9:1]

### Variante D

In einem 1 l-Rührwerk mit Tropftrichter, Thermometer und Rückflußkühler werden 110 g Toluol, 0,5 g Tetrabutylammoniumhydrogensulfat, 25 g H₃PO₄ 85 %ig und 60 g (0,32 mol) Diol-Gemisch (aus Beispiel 2) 10 Minuten unter Rückfluß gerührt, auf Raumtemperatur abgekühlt, die org. Phase abgetrennt, mit Sodalösung und Wasser neutralgewaschen, über Natrimsulfat getrocknet und als Lösungsmittel unter vermindertem Druck abdestilliert.

Es verbleiben 49,8 g Rohprodukt (racemisches cis/trans-Rosenoxid (**5a/b**)).

GC (Bedingungen siehe Beispiel 2): **5a** (63,7 %); **5b** (5,9 %); **B** (0,9 %)
Σ **5a + 5b** = 69,6 %
5a : 5b = 91:9

**Destillation über eine 30 cm Metallfüllkörperkolonne ergab 26,36 kg Kp**_{**20mbar**} = 78° - 80° C. Das entspricht einer Ausbeute von 58,9%.

GC (Bedingungen siehe Beispiel 2): **5a** (90,1 %); **5b** (7,5 %); **B** (0,76 %) **Σ 5a + b** = 97,6 %
5a: 5b = 92:8
- D 20/4 =: 0,8698
- n 20/D =: 1,4531
- [α] 20/D =: 0,0°

GC/MS: Bedingungen siehe Beispiel3
**5a** Rₜ = 15,57
MS (70 eV): m/e (%) = 154 (14M⁺), 139 (100), 85 (11), 83 (27), 69 (59), 67 (10), 55 (26), 41 (22), 39 (14).

**5b** Rₜ = 16,10
MS (70 eV): m/e (%) = 154 (11M⁺), 139 (100), 85 (10), 83 (25), 69 (58), 67 (10), 55 (26), 41 (19), 39 (13).

**5a** ¹³C-NMR (CDCl₃), Varian VXR-300: Δ[ppm]: 18.35, 22.34, 25.71 (CH₃), 34.44, 40.84, 67.87 (CH₂), 30.28, 74.61, 126.44 (CH), 134.94 (C).

**5b** ¹³C-NMR (CDCl₃), Varian VXR-300: Δ[ppm]: 18.29, 19.25, 25.79 (CH₃), 32.55, 38.21, 62.12 (CH₂), 24.97, 69.11, 125.37 (CH), 135.46 (C).

**B** Rₜ = 15,12
MS (70 eV): m/e (%) = 154 (36M⁺), 139 (100), 83 (24), 71 (53), 69 (60), 67 (25), 55 (67), 41 (77).

Rₜ = 15,20
MS (70 eV): m/e (%) = 154 (36M⁺), 139 (100), 83 (21), 71 (51), 69 (68), 67 (25), 55 (60), 41 (78).

### Beispiel 7

### Herstellung von (-)-3,7-Dimethyl-oct-5-en-1,7-diol und (-)-3,7-Dimethyl-oct-7-en-1,6-diol

112 g (0,72 mol) (-)-Citronellol [n 20/D = 1,4546; D 20/4 = 0,8735; [α] 20/D = - 23,1°]; (GC-Bedingungen siehe Beispiel 2, Rₜ= 6,1', 92,1 %) werden in einer Belichtungsapparatur (gemäß Beispiel 1) und anschließender Reduktion der Hydroperoxid-Lösung (gemäß Beispiel 2) in ein Gemisch von optisch aktiven (-)-3,7-Dimethyl-oct-5-en-1,7-diol und (-)-3,7-Dimethyl-oct-7-en-1,6-diol überführt. Es verbleiben 149 g Rohprodukt.

GC (Bedingungen gemäß Beispiel 2)
- (-)-3,7-Dimethyl-oct-5-en-1,7-diol: Rₜ = 7,6' (44,3 %)
- (-)-3,7-Dimethyl-oct-7-en-1,6-diol: Rₜ = 8,5' (43,9 %)

### Beispiel 8

### Herstellung von (-)-cis/trans-Rosenoxid

Unter den Verfahrensbedingungen gemäß Beispiel 3 (Variante A) werden aus 20 g (0,1 mol) Diol-Gemisch (aus Beispiel 7) 15,8 g Rohprodukt erhalten (GC-Bedingungen siehe Beispiel 2).
(-)-cis-Rosenoxid 64,4 %
(-)-trans-Rosenoxid 7,0 %
Σ cis+trans = 71,4 %
cis : trans = 90:10

Destillation über eine Drehbandkolonne ergibt 8,91 g Kp_{22mbar} = 80 - 81 °C, das entspricht einer Ausbeute von 59,1 %.
(-)-cis-Rosenoxid : (-)-trans-Rosenoxid = 9:1
- D 20/4 =: 0,8755
- n 20/D =: 1,4562
- [α] 20/D =: - 25.6°

GC/MS-, ¹³C-NMR- sowie ¹H-NMR-Daten stimmen mit den natürlichen Isolaten überein.

### Beispiel 9

### Herstellung eines Gemisches von (+)-3,7-Dimethyl-oct-5-en-1,7-diol und (+)-3,7-Dimethyl-oct-7-en-1,6-diol

112 g (0,72 mol) (+)-Citronellol [n 20/D = 1,4547, D 20/4 = 0,8732, [α] D/20 = + 3,5 °], GC-Bedingungen siehe Beispiel 2), Rₜ = 6,1' (92,4 %) werden in einer Belichtungsapparatur (gemäß Beispiel 1) und sich daran anschließender Na₂SO₃/H₂O-Reduktion der Hydroperoxid-Lösung (gemäß Beispiel 2) in ein Gemisch von optisch aktiven (+)-3,7-Dimethyl-oct-5-en-1,7-diol und (+)-3,7-Dimethyl-oct-7-en-1,6-diol überführt.

Es verbleiben 152 g Rohprodukt.

GC (Bedingungen siehe Beispiel 2)
- (+)-3,7-Dimethyl-oct-5-en-1,7-diol: Rₜ = 7,6' (45,1 %)
- (+)-3,7-Dimethyl-oct-7-en-1,6-diol: Rₜ = 8,5' (44,2 %)

### Beispiel 10

### Herstellung von (+)-cis/trans-Rosenoxid

Unter den Verfahrensbedingungen gemäß Beispiel 3 (Variante A) werden aus 20 g (0,1 mol) Diol-Gemisch (aus Beispiel 9) 15,9 g Rohprodukt erhalten.

GC (Bedingungen siehe Beispiel 2):
(+)-cis-Rosenoxid = 62,9 %; (+)-trans-Rosenoxid = 8,5 %
Σ cis + trans Rosenoxid = 71,4 %
cis : trans = 88:12

Eine Destillation über eine Drehbandkolonne ergibt 9,06 g Kp_{22mbar} = 80 - 81 °C. Das entspricht einer Ausbeute von 60,1 %.

GC (Bedingungen siehe Beispiel 2):
(+)-cis-Rosenoxid = 90,1 %;
(+)-trans-Rosenoxid = 8,2 %
Σ cis + trans Rosenoxid: 98,3 %
cis : trans = 91,5 : 8,5

- D 20/4 =: 0,8735
- n 20/D =: 1,4549
- [α] 20/D =: + 24,1°

GC/MS-Daten stimmen mit natürlichen Isolaten überein.

### Beispiel 11

### Herstellung von racemischem 3,7-Dimethyl-oct-7-en-1,6-diol (3b)

Nach der von L.-F. Tietze et al. [L.F. Tietze, Th. Eicher, 428 - 430, (1981), Jord. Stein Verlag, Stuttgart, New York] beschriebenen Synthese erhält man bei der Darstellung von Rosenoxid das 3,7-Dimethyl-oct-7-en-1,6-diol **3b** als höher siedende Komponente.

Ausgehend von dem in Beispiel 1 beschriebenen Gemisch zur Herstellung von racemischen Citronellyl-Hydroperoxid (**2a/b**), welches nach Reduktion unter den in Beispiel 2 genannten Bedingungen in ein Gemisch der racemischen Diole **3a/b** überführt wird, erhält man durch saure Cyclisierung nach der von L.-F. Tietze, Th. Eicher beschriebenen Methode ein Gemisch der beiden cis/trans-Rosenoxide (**5a/b**) sowie des nicht umgesetzten 3,7-Dimethyl-oct-7-en-1,6-diol (**3b**).

So erhält man ausgehend von 112 g (0,72 mol) Citronellol synthetisch nach Reduktion und Cyclisierung 105 g Rohprodukt.

GC (Bedingungen siehe Beispiel 2): **5a** (30,5 %); **5**b (9,3%); **3b** (40,1 %).

Destillation über eine 30 cm Metallfüllkörperkolonne ergab 36,8 g KP_{2mbar} 115 - 118 °C der Substanz **3b** (91 %).

### Beispiel 12 (nicht-erfindungsgemäßes Vergleichsbeispiel zu den erfindungsgemäßen Beispielen 13 und 14)

### Herstellung von racemischem cis/trans-Rosenoxid (5a + 5b) [9 : 1]

In einem 500 ml-Rührwerk mit Tropftrichter, Thermometer und Rückflußkühler werden 100 g Toluol, 3,5 g 50 %ige Schwefelsäure und 20 g (0,1 mol) 3,7-Dimethyl-oct-7-en-1,6-diol **3b** (aus Beispiel 11) 60 Minuten unter Rückfluß gerührt, auf Raumtemperatur abgekühlt, die organische Phase abgetrennt, mit Soda-Lösung und Wasser neutralgewaschen, über Natriumsulfat getrocknet und das Lösungsmittel unter vermindertem Druck abdestilliert. Dabei wird kein Allylether eingesetzt.

Es verbleiben 17,2 g Rohprodukt
GC (Bedingungen siehe Beispiel 2): **5a** (15,1 %); **5b** (1,6 %); **B** 1,8 %
Σ **5a + 5b** = 16,7 %
**5a : 5b** = 91:9

Destillation an einer Drehbandkolonne ergab 1,6 g KP_{22mbar} = 79 - 81 °C des racemischen Gemischs der Substanzen (5a/5b). Das entspricht einer Ausbeute von 10,1 %.

GC (Bedingungen siehe Beispiel 2): **5a** (90,1 %); **5b** (7,3 %); **B** = 1,8 % Σ **5a + 5b** = 97,4 %
**5a : 5b** = 92,5 : 7,5
- D 20/4 =: 0,8687
- n 20/D =: 1,4543
- [α]20/D =: 0,1°

### Beispiel 13

### Herstellung von racemischem cis/trans-Rosenoxid (5a + 5b) [9 : 1]

In einem 1 l-Rührwerk mit Tropftrichter, Thermometer, Rückflußkühler und Wasserabscheider werden 15,0 g (0,078 mol) 3,7-Dimethyl-oct-7-en-1,6-diol (**3b**) (aus Beispiel 11), 500 ml Cyclohexan, 1 g Filtrol® und 8,9 g (0,078 mol) 4-Methoxy-2-methyl-2-penten (als Beispiel eines Allylethers) über einen Zeitraum von 9 h unter Rückfluß gerührt. Während dieser Zeit wurden insgesamt 0,7 ml H₂O abgespalten.

Nach dem Abkühlen auf Raumtemperatur wird das Filtrol® abfiltriert und die org. Phase mit Sodalösung und Wasser neutralgewaschen, über Na₂SO₄ getrocknet und das Lösungsmittel unter vermindertem Druck abdestilliert. Es verbleiben 12 g Rohprodukt.

GC (Bedingungen siehe Beispiel 2): **5a** (38,4 %); **5b** (4,3 %); **B** = 1,6 %
Σ **5a + 5b** = 42,7 %
**5a : 5b** = 90:10

Destillation an einer Drehbandkolonne ergab 3,15 g KP_{23mbar} = 81 - 82,5 °C. Das entspricht einer Ausbeute von 26,3 %.

GC (Bedingungen siehe Beispiel 2): **5a** (90,3 %); **5b** (7,8 %); **B** = 1,6 %
Σ **5a + 5b** = 98,1 %
**5a : 5b** = 90:10
- D 20/4 =: 0,8701
- n 20/D =: 1,4536
- [α]20/D =: 0,1°

### Beispiel 14

### Herstellung von racemischem cis/trans-Rosenoxid (5a + 5b) [92 : 8]

In einem 1 l-Rührwerk mit Thermometer, Rückflußkühler und Wasserabscheider werden 15 (0,078 mol) 3,7-Dimethyl-oct-7-en-1,6-diol (**3b**) (aus Beispiel 11), 500 ml Cyclohexan, 1 g Filtrol® und 12 g (0,078 mol) cis/trans-Rosenoxid **5a/b** (aus Beispiel 3) über einen Zeitraum von 10 h unter Rückfluß gerührt (Anmerkung: das in äquimolarer Menge neben dem Diol **3b** eingesetzte cis/trans-Rosenoxid **5a/b** ist ein besonders geeigneter Allylether). Während dieser Zeit wurden 1,1 ml H₂O abgespalten. Nach dem Abkühlen auf Raumtemperatur wird das Filtrol® abfiltriert und die organische Phase mit Sodalösung und Wasser neutral gewaschen, über Na₂SO₄ getrocknet und das Lösungsmittel unter vermindertem Druck abdestilliert. Es verbleiben 25 g Rohprodukt.

GC (Bedingungen siehe Beispiel 2): **5a** (70,3 %); **5b** (5,8 %); **B** = (1,0 %)
Σ **5a + 5b** = 76,1 %
**5a : 5b** = 92:8

Destillation an einer Drehbandkolonne ergab 18,1 g
KP_{22mbar} = 79 - 81 °C. Das entspricht einer Ausbeute von 6,1 g = 50,8 %.

GC (Bedingungen siehe Beispiel 2): **5a** (90,1 %); **5b** (7,6 %); **B** = (0,9 %).
Σ **5a + 5b** = 97,7 %
**5a : 5b** = 92:8
- D 20/4 =: 0,8700
- n 20/D =: 1,4534
- [α]20/D =: 0,1°

Ein Vergleich der Beispiele 12, 13 und 14 zeigt eine deutliche Ausbeute-Abstufung zwischen dem nicht-erfindungsgemäßen (Vergleichs-)Beispiel 12 und den erfindungsgemäßen Beispielen 13 und 14 sowie eine ebenfalls deutliche Abstufung zwischen den erfindungsgemäßen Beispielen 13 und 14. Rosenoxid ist demnach als Allylether-Komponente besonders gut geeignet.

Den Beispielen 11 - 14 entsprechende Beispiele wurden mit den jeweils entsprechenden optisch aktiven Edukt-Spezies (Citronellol, Citronellyl-Hydroperoxid, Diol **3b**) durchgeführt. Es ergaben sich die jeweils analogen Resultate.

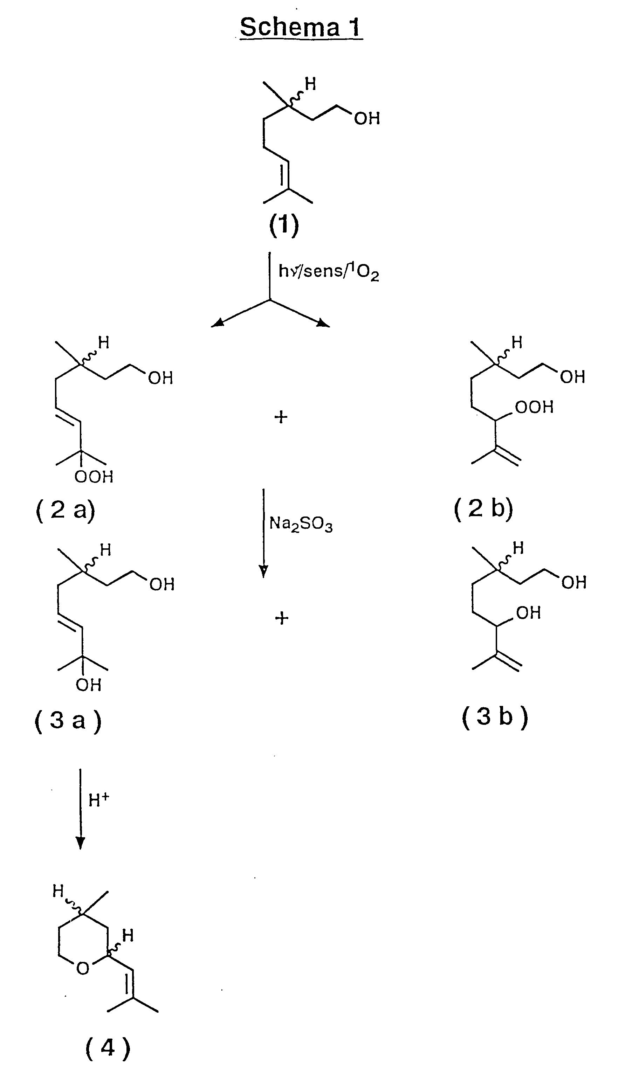

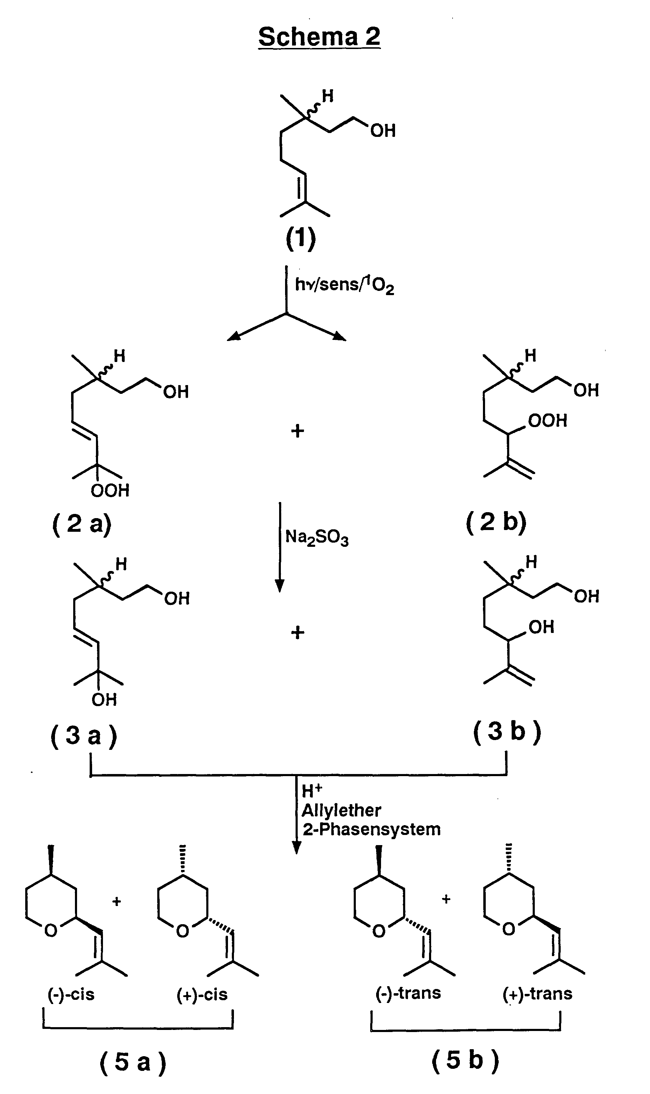

## Patentansprüche

1. Verfahren zur Herstellung von Rosenoxid der allgemeinen Formel A, **dadurch gekennzeichnet, daß** 3,7-Dimethyl-oct-7-en-1,6-diol (**3b**) in Anwesenheit eines gegebenenfalls in situ gebildeten Allylethers mit Säure behandelt wird, wobei die Säurebehandlung in einem Zweiphasensystem flüssig/ flüssig unter den Bedingungen einer Phasentransferkatalyse oder in einem Zweiphasensystem flüssig/fest mittels einer an einer Festsubstanz gebundenen Säure erfolgt.

2. Verfahren nach Anspruch 1, **dadurch gekennzeichnet, daß** das 3,7-Dimethyl-oct-7-en-1,6-diol (**3b**) im Gemisch mit 3,7-Dimethyl-oct-7-en-1,7-diol (**3a**) der Säurebehandlung unterzogen wird.

3. Verfahren nach Anspruch 1 oder 2, **dadurch gekennzeichnet, daß** es sich bei dem Allylether um ein Rosenoxid handelt, das mit dem 3,7-Dimethyl-oct-7-en-1,6-diol (**3b**) oder dem Diol-Gemisch (**3a + 3b**) vor oder während der Säurebehandlung vermischt wird und/oder sich während der Säurebehandlung in situ aus dem gegebenenfalls anwesenden 3,7-Dimethyl-oct-7-en-1,7-diol (**3a**) bildet.

4. Verfahren nach einem der vorangehenden Ansprüche, **dadurch gekennzeichnet, daß** zur Säurebehandlung des Diol-Gemisches (**3a + 3b**) oder des Diols (**3b**) Schwefelsäure, Phosphorsäure, eine säureaktivierte Bleicherde oder eine Heteropolywolframsäure wie Wolframatokieselsäure oder Wolframatophosphorsäure eingesetzt wird.

5. Verfahren nach einem der Ansprüche 2 - 4, **dadurch gekennzeichnet, daß** ein Gemisch der beiden Diole (**3a**) und (**3b**) eingesetzt wird, welches aus Citronellol der allgemeinen Formel (1) z.B. durch photosensibilisierte Singulett-Sauerstoff-Oxidation und anschließende Reduktion der erhaltenen Hydroperoxide (**2a + 2b**) gewonnen ist.

6. Verfahren nach einem der vorangehenden Ansprüche, **dadurch gekennzeichnet, daß** das Diolgemisch (**3a + 3b**) oder das Diol (**3b**) in einem aprotischen Lösungsmittel gelöst wird.

7. Verfahren nach einem der vorangehenden Ansprüche, **dadurch gekennzeichnet, daß** eine Stoffmenge des 3,7-Dimethyl-oct-7-en-1,6-diols (**3b**), gegebenfalls im Gemisch mit dem Diol (**3a**), mit einer gleichen, größeren oder nur geringfügig geringeren Stoffmenge Rosenoxid vermischt wird.

8. Verfahren nach einem der vorangehenden Ansprüche, **dadurch gekennzeichnet, daß** die Reaktion unter Zusatz eines Phasentransferkatalysators wie z.B. Aliquat, Tetrabutylammonium-chlorid, -bromid oder hydrogensulfat in Konzentrationen von 0,1 Gew.-% bis 10 Gew.-%, vorzugsweise von 4-6 Gew.-%, bezogen auf die Menge des eingesetzten Diolgemischs (**3a + 3b**) oder Diols (**3b**), durchgeführt wird.

9. Verfahren nach einem der vorangehenden Ansprüche, **dadurch gekennzeichnet, daß** zur Säurebehandlung des Diol-Gemisches (**3a + 3b**) oder des Diols (**3b**) eine handelsübliche aktivierte Bleicherde wie Montmorillonit K10, Montmorillonit KSF, Filtrol, Katalysator KS, Tonsil Optimum in einer Konzentration von 0,5 bis 40 Gew.%, vorzugsweise von 1 - 20 Gew%, bezogen auf die Menge des eingesetzten Diolgemischs (**3a + 3b**) oder Diols (**3b**), verwendet wird.

## Claims

1. Process for the preparation of rose oxide of the general formula A, **characterised in that** 3,7-dimethyl-oct-7-ene 1,6-diol (**3b**) is treated with acid in the presence of an allyl ether that is optionally formed in situ, the acid treatment taking place in a liquid/liquid two-phase system under the conditions of a phase transfer catalysis or in a liquid/solid two-phase system by means of an acid bound to a solid substance.

2. Process according to Claim 1, **characterised in that** the 3,7-dimethyl-oct-7-ene 1,6 diol (**3b**) is subjected to the acid treatment in a mixture with 3,7-dimethyl-oct-7-ene 1,7 diol (**3a**).

3. Process according to Claim 1 or 2, **characterised in that** the allyl ether is a rose oxide that is mixed with the 3,7-dimethyl-oct-7-ene 1,6 diol (**3b**) or the diol mixture (**3a+3b**) before or during the acid treatment and/or forms in situ during the acid treatment from the 3,7-dimethyl-oct-7-ene 1,7 diol (**3a**) that may be present.

4. Process according to one of the preceding claims, **characterised in that** sulphuric acid, phosphoric acid, an acid-activated Fuller's earth or a heteropolytungstic acid, such as tungstosilicic acid or tungstophosphoric acid, is used for the acid treatment of the diol mixture (**3a+3b**) or of the diol (**3b**).

5. Process according to one of Claims 2 - 4, **characterised in that** a mixture of the two diols (**3a**) and (**3b**) is used which has been obtained from citronellol of the general formula (1), for example by photosensitised singlet oxygen oxidation and subsequent reduction of the hydroperoxides (**2a+2b**) obtained.

6. Process according to one of the preceding claims, **characterised in that** the diol mixture (**3a+3b**) or the diol (**3b**) is dissolved in an aprotic solvent.

7. Process according to one of the preceding claims, **characterised in that** an amount of the substance 3,7-dimethyl-oct-7-ene 1,6 diol (**3b**), optionally in a mixture with the diol (**3a**), is mixed with the same amount, a larger amount or an only slightly smaller amount of the substance rose oxide.

8. Process according to one of the preceding claims, **characterised in that** the reaction is carried out with the addition of a phase transfer catalyst, such as, for example, Aliquat, tetrabutylammonium chloride, tetrabuylammonium bromide or [lacuna] hydrogen sulphate in concentrations of 0.1 % (m/m) to 10 % (m/m), preferably of 4 - 6 % (m/m), based on the amount of the diol mixture (**3a+3b**) or diol (**3b**) used.

9. Process according to one of the preceding claims, **characterised in that** for the acid treatment of the diol mixture (**3a+3b**) or of the diol (**3b**) a commercially available activated Fuller's earth, such as montmorillonite K10, montmorillonite KSF, Filtrol, catalyst KS or Tonsil Optimum, is used in a concentration of 0.5 - 40 % (m/m), preferably of 1 - 20 % (m/m), based on the amount of the diol mixture (**3a+3b**) or diol (**3b**) used.

## Revendications

1. Procédé pour la préparation de l'oxyde de rose, selon la formule générale A, **caractérisé en ce que** du 3,7-diméthyl-oct-7-en-1,6-diol (3b) est traité à l'acide, en présence d'un éther allylique, formé le cas échéant in situ, le traitement à l'acide étant effectué dans un système à deux phases liquide/liquide, dans des conditions d'une catalyse par transfert de phase ou dans un système à deux phases liquide/solide, au moyen d'un acide lié à une substance solide.

2. Procédé selon la revendication 1, **caractérisé en ce que** le 3,7-diméthyl-oct-7-en-1,6-diol (3b) est soumis au traitement à l'acide, en mélange avec le 3,7-diméthyl-oct-7-en-1,7-diol (3a).

3. Procédé selon la revendication 1 ou 2, **caractérisé en ce que** l'éther allylique est un oxyde de rose qui est mélangé au 3,7-diméthyl-oct-7-en-1,6-diol (3b) ou au mélange de diols (3a + 3b) avant ou pendant le traitement à l'acide et/ou **en ce qu'**il se forme au cours du traitement à l'acide in situ, à partir du 3,7-diméthyl-oct-7-en-1,7-diol (3a) présent le cas échéant.

4. Procédé selon l'une quelconque des revendications précédentes, **caractérisé en ce que**, pour le traitement à l'acide du mélange de diols (3a + 3b) ou du diol (3b), on utilise de l'acide sulfurique, de l'acide phosphorique, une argile décolorante activée par acide ou un hétéro-polyacide tungstique, comme l'acide silicique de tungstate ou l'acide phosphorique de tungstate.

5. Procédé selon l'une quelconque des revendications 2 à 4, **caractérisé en ce qu'**on utilise un mélange des deux diols (3a) et (3b), lequel est obtenu à partir d'huile de citronnelle de la formule générale (1), par exemple par oxydation photosensibilisée oxygène - singulet, suivie d'une réduction des hydroperoxydes (2a + 2b) ainsi obtenus.

6. Procédé selon l'une quelconque des revendications précédentes, **caractérisé en ce que** le mélange de diols (3a + 3b) ou le diol (3b) est dilué dans un diluant aprotique.

7. Procédé selon l'une quelconque des revendications précédentes, **caractérisé en ce qu'**une quantité de la substance 3,7-diméthyl-oct-7-en-1,6-diol (3b), le cas échéant mélangée au diol (3a) est mélangée à une quantité soit égale, soit plus importante, soit à peine plus faible d'oxyde de rose.

8. Procédé selon l'une quelconque des revendications précédentes, **caractérisé en ce que** la réaction est réalisée sous adjonction d'un catalyseur par transfert de phase, comme par exemple l'aliquat, le chlorure de tétrabutylammonium, le bromure de tétrabutylammonium ou du sulfate d'hydrogène, à des concentrations de 0,1 % en poids jusqu'à 10 % en poids, de préférence de 4 à 6 % en poids, en fonction de la quantité de mélange des diols (3a + 3b) ou du diol (3b) mis en oeuvre.

9. Procédé selon l'une quelconque des revendications précédentes, **caractérisé en ce que**, pour le traitement à l'acide du mélange de diols (3a + 3b) ou du diol (3b), une argile décolorante activée du commerce, comme la montmorillonite K10, la montmorillonite KSF, le Filtrol, le catalyseur KS, le Tonsil Optimum, est utilisée dans une concentration de 0,5 à 40 % en poids, de préférence de 1 à 20 % en poids, en fonction de la quantité de mélange de diols (3a + 3b) ou du diol (3b) mis en oeuvre.
